# EUROPÄISCHE PATENTSCHRIFT

(11) **EP 0 904 151 B1**
(45) Veröffentlichungstag und Bekanntmachung des Hinweises auf die Patenterteilung: **08.08.2001**
(21) Anmeldenummer: 97925025.5
(22) Anmeldetag: 30.05.1997
(51) Int. Cl.: B01J 29/04, C07D 301/10

(54) **OXIDATIONSKATALYSATOR UND VERFAHREN ZUR HERSTELLUNG VON EPOXIDEN AUS OLEFINEN, WASSERSTOFF UND SAUERSTOFF UNTER VERWENDUNG DES OXIDATIONSKATALYSATORS**
OXIDATION CATALYST AND PROCESS FOR THE PRODUCTION OF EPOXIDES FROM OLEFINES, HYDROGEN AND OXYGEN USING SAID OXIDATION CATALYST
CATALYSEUR D'OXYDATION ET PROCEDE DE PRODUCTION D'EPOXYDES A PARTIR D'OLEFINES, D'HYDROGENE ET D'OXYGENE A L'AIDE DUDIT CATALYSEUR D'OXYDATION

(30) Priorität: 13.06.1996 DE 19623609
(43) Veröffentlichungstag der Anmeldung: 31.03.1999
(73) Patentinhaber: BASF AKTIENGESELLSCHAFT, 67056 Ludwigshafen (DE)
(72) Erfinder: GROSCH, Georg, Heinrich, D-67098 Bad Dürkheim (DE); MÜLLER, Ulrich, D-67434 Neustadt (DE); SCHULZ, Michael, D-67067 Ludwigshafen (DE); RIEBER, Norbert, D-68259 Mannheim (DE); WÜRZ, Harald, D-67487 Maikammer (DE)
(86) Internationale Anmeldenummer: EP9702821
(87) Internationale Veröffentlichungsnummer: WO9747386

(56) Entgegenhaltungen:
- EP-A- 0 229 295
- EP-A- 0 325 053
- EP-A- 0 326 759
- EP-A- 0 640 598
- DE-A- 4 425 672
- DE-A- 4 435 239

## Beschreibung

Die vorliegende Erfindung betrifft einen neuen Oxidationskatalysator auf Basis von bestimmten Titan-' oder Vanadiumsilikaliten mit Zeolith-Struktur und mit einem Gehalt an Edelmetallen sowie ein Verfahren zur Herstellung von Epoxiden aus Olefinen, Wasserstoff und Sauerstoff unter Verwendung dieses Oxidationskatalysators.

Edelmetallhaltige Oxidationskatalysatoren auf Basis von Titan- oder Vanadiumsilikaliten mit Zeolith-Struktur sowie ein Verfahren zur Herstellung von Epoxiden aus Olefinen, Wasserstoff und Sauerstoff unter Verwendung dieser Oxidationskatalysatoren sind aus der WO-A 96/02323 bekannt. Dort werden die Katalysatoren als kristallines Pulver eingesetzt.

In EP-A 640 598 und EP-A 325 053 werden Oxidationskatalysatoren auf Basis von Titansilikaliten mit Zeolith-Struktur und einem Gehalt an Edelmetallen beschrieben, welche zur Herstellung von Epoxiden aus Olefinen verwendet werden können. Diese Katalysatoren sind zu Formkörpern, z.B. zu Pellets, geformt.

Derartige Oxidationskatalysatoren des Standes der Technik weisen jedoch Nachteile auf. Bei Verwendung von nicht geformten Epoxidierungskatalysatoren sind diese zu feinkörnig, so daß sie mechanische Probleme, beispielsweise bei deren Abtrennung, verursachen.

Aufgabe der vorliegenden Erfindung war es daher, Epoxidierungskatalysatoren, bereitzustellen, welche die Nachteile des Standes der Technik nicht mehr aufweisen.

Demgemäß wurden ein Oxidationskatalysator auf Basis von Titan- oder Vanadiumsilikaliten mit Zeolith-Struktur mit röntgenographischer Zuordnung zur MFI-, MEL- oder MFI/MEL-Mischstruktur und mit einem Gehalt von 0,01 bis 30 Gew.-% an einem oder mehreren Edelmetallen aus der Gruppe Ruthenium, Rhodium, Palladium, Osmium, Iridium, Platin, Rhenium, Gold und Silber gefunden, welcher dadurch gekennzeichnet ist, daß der Oxidationskatalysator einem verfestigenden Formgebungsprozess durch Extrudieren zu Strängen unterworfen worden ist.

Der verfestigende Formgebungsprozess erfolgt durch Extrusion in üblichen Extrudern, beispielsweise zu Strängen mit einem Durchmesser von üblicherweise 1 bis 10 mm, insbesondere 2 bis 5 mm. Werden Bindemittel und/oder Hilfsmittel benötigt, ist der Extrusion zweckmäßigerweise ein Mischungs- oder Knetprozeß vorgeschaltet. Gegebenenfalls erfolgt nach der Extrusion noch ein Kalzinierungs- schritt. Die erhaltenen Stränge werden gewünschtenfalls zerkleinert, vorzugsweise zu Granulat oder Splitt mit einem Partikeldurchmesser von 0,5 bis 5 mm, insbesondere 0,5 bis 2 mm. Dieses Granulat oder dieser Splitt und auch auf anderem Wege erzeugte Katalysatorformkörper enthalten praktisch keine feinkörnigeren Anteile als solche mit 0,5 mm Mindestpartikeldurchmesser.

In einer bevorzugten Ausführungsform enthält der erfindungsgemäße geformte Oxidationskatalysator bis zu 10 Gew.-% Bindemittel, bezogen auf die Gesamtmasse des Katalysators. Besonders bevorzugte Bindemittelgehalte sind 0,1 bis 7 Gew.-%, insbesondere 1 bis 5 Gew.-%. Als Bindemittel eignen sich im Prinzip alle für derartige Zwecke eingesetzte Verbindungen; bevorzugt werden Verbindungen, insbesondere Oxide, des Siliciums, Aluminiums, Bors, Phosphors, Zirkoniums und/oder Titans. Von besonderem Interesse als Bindemittel ist Siliciumdioxid, wobei das SiO₂ als Kieselsol oder in Form von Tetraalkoxysilanen in den Formgebungsschritt eingebracht werden kann. Auch als Bindemittel verwendbar sind Oxide des Magnesiums und Berylliums sowie Tone, z.B. Montmorillonite, Kaoline, Bentonite, Halloysite, Dickite, Nacrite und Ananxite.

Als Hilfsmittel für die verfestigenden Formgebungsprozesse sind beispielsweise Verstrangungshilfsmittel für die Extrusion zu nennen, ein übliches Verstrangungshilfsmittel ist Methylcellulose. Derartige Mittel werden in der Regel in einem nachfolgenden Kalzinierungsschritt vollständig verbrannt.

Die so hergestellten geformten Oxidationskatalysatoren weisen eine hohe massenspezifische Aktivität und eine für alle Umsetzungsfahrweisen und Reaktortypen ausreichende Härte und Abriebfestigkeit auf.

Die geformten Oxidationskatalysatoren basieren auf bestimmten Titan- oder Vanadiumsilikaliten mit Zeolith-Struktur. Zeolithe sind bekanntermaßen kristalline Alumosilikate mit geordneten Kanal- und Käfigstrukturen, deren Porenöffnungen im Bereich von Mikroporen, die kleiner als 0,9 nm sind, liegen. Das Netzwerk solcher Zeolithe ist aufgebaut aus SiO₄- und AlO₄-Tetraedern, die über gemeinsame Sauerstoffbrücken verbunden sind. Eine Übersicht der bekannten Strukturen findet sich beispielsweise bei W.M. Meier und D.H. Olson, "Atlas of Zeolite Structure Types", Butterworth, 2nd Ed., London 1987.

Es sind nun auch Zeolithe bekannt, die kein Aluminium enthalten und bei denen im Silikatgitter anstelle des Si(IV) teilweise Titan als Ti(IV) steht. Diese Titanzeolithe, insbesondere solche mit einer Kristallstruktur vom MFI-Typ, sowie Möglichkeiten zu ihrer Herstellung sind beschrieben, beispielsweise in der EP-A 311 983 oder der EP-A 405 978. Außer Silizium und Titan können solche Materialien auch zusätzliche Elemente wie Aluminium, Zirkonium, Zinn, Eisen, Kobalt, Nickel, Gallium, Bor oder geringe Mengen an Fluor enthalten.

Im erfindungsgemäßen Oxidationskatalysator kann das Titan des Zeoliths teilweise oder vollständig durch Vanadium ersetzt sein. Das molare Verhältnis von Titan und/oder Vanadium zur Summe aus Silicium plus Titan und/oder Vanadium liegt in der Regel im Bereich von 0,01:1 bis 0,1:1.

Titanzeolithe mit MFI-Struktur sind dafür bekannt, daß sie über ein bestimmtes Muster bei der Bestimmung ihrer Röntgenbeugungsaufnahmen sowie zusätzlich über eine Gerüstschwingungsbande im Infrarotbereich (IR) bei etwa 960 cm⁻¹ identifiziert werden können und sich damit von Alkalimetalltitanaten oder kristallinen und amorphen TiO₂-Phasen unterscheiden.

Typischerweise stellt man die genannten Titan- und auch Vanadiumzeolithe dadurch her, daß man eine wäßrige Mischung aus einer SiO₂-Quelle, einer Titan- bzw. Vanadium-Quelle wie Titandioxid bzw. einem entsprechenden Vanadiumoxid und einer stickstoffhaltigen organischen Base ("Schablonen-Verbindung"), z.B. Tetrapropylammoniumhydroxid, gegebenenfalls noch unter Hinzufügen von Alkalimetallverbindungen, in einem Druckbehälter unter erhöhter Temperatur im Zeitraum mehrerer Stunden oder einiger Tage umsetzt, wobei das kristalline Produkt entsteht. Dieses wird abfiltriert, gewaschen, getrocknet und zur Entfernung der organischen Stickstoffbase bei erhöhter Temperatur gebrannt. In dem so erhaltenen Pulver liegt das Titan bzw. das Vanadium zumindest teilweise innerhalb des Zeolithgerüsts in wechselnden Anteilen mit vier-, fünf- oder sechsfacher Koordination vor. Zur Verbesserung des katalytischen Verhaltens kann sich noch eine mehrmalige Waschbehandlung mit schwefelsaurer Wasserstoffperoxidlösung anschließen, worauf das Titan- bzw. Vanadiumzeolith-Pulver erneut getrocknet und gebrannt werden muß; daran kann sich eine Behandlung mit Alkalimetallverbindungen anschließen, um den Zeolith von der H-Form in die Kation-Form zu überführen. Das so hergestellte Titan- bzw. Vanadiumzeolith-Pulver wird dann im Sinne der vorliegenden Erfindung wie oben beschrieben geformt.

Die eingesetzten Titan- oder Vanadiumzeolithe sind solche mit Pentasil-Zeolith-Struktur mit röntgenographischer Zuordnung zur MFI-, MEL- oder MFI/MEL-Mischstruktur. Zeolithe dieses Typs sind beispielsweise in W.M. Meier und D.H. Olson, "Atlas of Zeolite Structure Types", Butterworths, 2nd Ed., London 1987, beschrieben.

Der erfindungsgemäße Oxidationskatalysator weist einen Gehalt von 0,01 bis 30 Gew.-%, insbesondere 0,05 bis 15 Gew.-%, vor allem 0,1 bis 8 Gew.-%, jeweils bezogen auf die Menge der Titan- oder Vanadium-Zeolithe, der genannten Edelmetallen auf. Hierbei wird Palladium besonders bevorzugt. Die Edelmetalle können auf den Katalysator in Form geeigneter Edelmetallkomponenten, beispielsweise in Form von wasserlöslichen Salzen, vor, während oder im Anschluß an den verfestigenden Formgebungsschritt aufgebracht werden.

In vielen Fällen ist es jedoch am günstigsten, die Edelmetallkomponenten erst nach dem Formgebungsschritt auf die Katalysatorformkörper zu bringen, besonders dann, wenn eine Hochtemperaturbehandlung des edelmetallhaltigen Katalysators unerwünscht ist. Die Edelmetallkomponenten können insbesondere durch Ionenaustausch, Imprägnierung oder Aufsprühen auf den geformten Katalysator gebracht werden. Das Aufbringen kann mittels organischer Lösungsmittel, wäßriger ammoniakalischer Lösungen oder überkritischer Phasen wie etwa Kohlendioxid erfolgen.

Durch den Einsatz dieser vorgenannten Methoden können durchaus verschiedenartige edelmetallhaltige Katalysatoren erzeugt werden. so kann durch Aufsprühen der Edelmetallösung auf die Katalysatorformteile ein Art Schalenkatalysator erzeugt werden. Die Dicke dieser edelmetallhaltigen Schale läßt sich durch Imprägnieren deutlich vergrößern, währen beim Ionenaustausch die Katalysatorpartikel weitgehend gleichmäßig über den Formkörperquerschnitt mit Edelmetall belegt werden.

Der erfindungsgemäße geformte Oxidationskatalysator eignet sich in hervorragender Weise zur Epoxidierung von Olefinen mittels Wasserstoff und Sauerstoff. Daher ist auch Gegenstand der vorliegenden Erfindung ein Verfahren zur Herstellung von Epoxiden aus Olefinen, Wasserstoff und Sauerstoff, welches dadurch gekennzeichnet ist, daß man die Olefine heterogenkatalytisch unter Verwendung eines erfindungsgemäßen Oxidationskatalysators umsetzt.

Das erfindungsgemäße Verfahren zur Herstellung von Epoxiden kann im Prinzip mit allen üblichen Umsetzungsfahrweisen und in allen üblichen Reaktortypen durchgeführt werden, beispielsweise in Suspensionsfahrweise oder in einer Festbettanordnung. Man kann kontinuierlich oder diskontinuierlich arbeiten. Vorzugsweise wird die Epoxidierung jedoch in einer Festbettapparatur durchgeführt.

Abhängig vom umzusetzenden Olefin kann die erfindungsgemäße Epoxidierung in flüssiger Phase, in der Gasphase oder auch in überkritischer Phase durchgeführt werden, wobei eine Umsetzung der Olefine mit dem Wasserstoff/Sauerstoff-Gasgemisch in einer Flüssigphasen- oder einer Gasphasen-Fahrweise bevorzugt wird.

Wird die erfindungsgemäße Epoxidierung in flüssiger Phase vorgenommen, arbeitet man vorteilhafterweise bei einem Druck von 1 bis 10 bar und in Gegenwart von Lösungsmitteln. Als Lösungsmittel eigenen sich Alkohole, z.B. Methanol, Ethanol, iso-Propanol oder tert.-Butanol oder Mischungen hieraus, und insbesondere Wasser. Mann kann auch Mischungen der genannten Alkohole mit Wasser einsetzen. In bestimmten Fällen bewirkt die Verwendung von Wasser oder wasserhaltigen Lösungsmittelsystemen eine deutliche Selektivitätssteigerung des gewünschten Epoxids gegenüber den reinen Alkoholen als Lösungsmittel.

Die erfindungsgemäßen Epoxidierung wird in der Regel bei Temperaturen von -20 bis 70°C, insbesondere -5 bis 50°C, vorgenommen. Das Molverhältnis von Wasserstoff zu Sauerstoff kann üblicherweise im Bereich H₂:O₂ = 1:10 bis 1:1 variiert werden und ist besonders günstig bei 1:5 bis 1:1. Das molare Verhältnis von Sauerstoff zu Olefin liegt hierbei in der Regel bei 1:4 bis 1:10, vorzugsweise 1:5 bis 1:7. Als Trägergas kann ein beliebiges Inertgas zugefahren werden, insbesondere eignet sich Stickstoff.

Das eingesetzte Olefin kann eine beliebige organische Verbindung sein, die mindestens eine ethylenisch ungesättigte Doppelbindung enthält. Sie kann aliphatischer, aromatischer oder cycloaliphatischer Natur sein, sie kann aus einer linearen oder einer verzweigten Struktur bestehen. Vorzugsweise enthält das Olefin 2 bis 30 C-Atome. Mehr als eine ethylenisch ungesättigte Doppelbindung kann vorhanden sein, so etwa in Dienen oder Trienen. Das Olefin kann zusätzlich funktionelle Gruppe wie Halogenatome, Carboxylgruppen, Carbonesterfunktionen, Hydroxylgruppen, Etherbrücken, Sulfidbrücken, Carbonylfunktionen, Cyanogruppen, Nitrogruppen oder Aminogruppen enthalten.

Typische Beispiele für derartige Olefine sind Ethylen, Propen, 1-Buten, cis- und trans-2-Buten, 1,3-Butadien, Pentene, Isopren, Hexene, Octene, Nonene, Decene, Undecene, Dodecene, Cyclopenten, Cyclohexen, Dicyclopentadien, Methylencyclopropan, Vinylcyclohexan, Vinylcyclohexen, Allylchlorid, Acrylsäure, Methacrylsäure, Crotonsäure, Vinylessigsäure, Allylalkohol, Alkylacrylate, Alkylmethacrylate, Ölsäure, Linolsäure, Linolensäure, Ester und Glyceride derartiger ungesättigter Fettsäuren, Styrol, α-Methylstyrol, Divinylbenzol, Inden und Stilben. Auch Mischungen der genannten Olefine können nach dem erfindungsgemäßen Verfahren epoxidiert werden.

Das erfindungsgemäße Verfahren eignet sich in besonderem Maße für die Epoxidierung von Propen zu Propylenoxid.

Die erfindungsgemäßen geformten Oxidationskatalysatoren, weisen eine Reihe von Vorteilen auf. Wie schon erwähnt besitzen die Oxidationskatalysatoren eine hohe massenspezifische Aktivität, welche sich auch im Laufe der Zeit nicht wesentlich vermindert, und eine ausreichende Härte und Abreibfestigkeit, was sie insbesondere für den Einsatz in Festbettapparaturen interessant macht. Dadurch, daß die Katalysatorformkörper keine klein- und kleinstteiligen Anteile besitzen, welche durch Rückhaltungseffekte negative Einflüsse ausüben können, ist das Neben- und Folgeproduktspektrum bei der Epoxidierung gering und eine damit verbundene Aktivitätsminderung über die Zeit praktisch nicht feststellbar.

Auch von Vorteil ist der nur geringe Anteil an benötigtem Bindemittel, d.h. maximal 10 Gew.-%, im geformten Oxidationskatalysator, üblicherweise enthalten solche Katalysatoren bis zu 20 Gew.-% an Bindemittel. Derart hohe Bindemittelgehalte beeinträchtigen naturgemäß die Aktivität des Katalysators.

Die nachfolgenden Beispiele sollen die Herstellung der erfindungsgemäßen Oxidationskatalysatoren und die erfindungsgemäße Epoxidierung erläutern, ohne daß dadurch jedoch eine Beschränkung zu verstehen wäre.

### Beispiel 1

In einem Vierhalskolben (2 l Inhalt) wurden 455 g Tetraethylorthosilikat vorgelegt und aus einem Tropftrichter innerhalb von 30 min mit 15 g Tetraisopropylorthotitanat unter Rühren (250 U/min, Blattrührer) versetzt. Es bildete sich eine farblose, klare Mischung. Abschließend versetzte man mit 800 g einer 20 gew.-%igen Tetrapropylammoniumhydroxid-Lösung (Alkaligehalt < 10 ppm) und rührte noch eine Stunde nach. Bei 90°C bis 100°C wurde das aus der Hydrolyse gebildete Alkoholgemisch (ca. 450 g) abdestilliert. Man füllte mit 1,5 l deionisiertem Wasser auf und gab das mittlerweile leicht opake Sol in einen 2,5 l fassenden Rührautoklaven aus Edelstahl.

Mit einer Heizrate von 3°/min wurde der verschlossene Autoklav (Ankerrührer, 200 U/min) auf eine Reaktionstemperatur von 175°C gebracht. Nach 92 Stunden war die Reaktion beendet. Das erkaltete Reaktionsgemisch (weiße Suspension) wurde abzentrifugiert und mehrfach mit Wasser neutral gewaschen. Der erhaltene Feststoff wurde bei 110°C innerhalb von 24 Stunden getrocknet (Auswaage 149 g).

Abschließend wurde unter Luft bei 550°C in 5 Stunden das im Zeolithen noch verbliebene Templat abgebrannt (Kalzinierungsverlust: 14 Gew.-%).

Das reinweiße Produkt hatte nach naßchemischer Analyse einen Ti-Gehalt von 1,5 Gew.-% und einen Gehalt an Restalkali unterhalb 100 ppm. Die Ausbeute auf eingesetztes SiO₂ betrug 97 %. Die Kristallite hatten eine Größe von 0,05 - 0,25 µm und das Produkt zeigte im IR eine typische Bande bei ca. 960 cm⁻¹.

### Beispiel 2

1000 g Titansilikalit aus Beispiel 1 wurden in einer Mischung aus 6 l einer 5 gew.-%igen Schwefelsäure und 600 g 30 gew.-%iger Wasserstoffperoxidlösung suspendiert und bei 80°C 2 h lang gerührt. Danach wurde der so behandelte Titansilikat abgesaugt und weitere dreimal wie beschrieben behandelt. Danach wurde der Titansilikalit in 6 l Wasser suspendiert, bei 80°C 2 h lang gerührt und abgesaugt. Dieser Vorgang wurde einmal wiederholt. Danach wurde der so behandelte Festkörper bei 150°C getrocknet und anschließend bei 500°C 5 h lang unter Luft kalziniert.

### Beispiel 3

950g Titansilikalit aus Beispiel 2 wurden in 6 l einer 1 gew.-%igen Natriumacetatlösung in Wasser suspendiert und für 20 min unter Rückfluß gekocht, danach wurde der Titansilikalit abgesaugt. Dieser Vorgang wurde noch zweimal wiederholt. Anschließend wurde der so behandelte Titansilikalit in 6 l Wasser suspendiert, 30 min unter Rückfluß gekocht und abgesaugt. Auch dieser Vorgang wurde wiederholt. Der Titansilikalit wurde dann bei 150°C getrocknet und bei 500°C kalziniert.

### Beispiel 4

100 g Titansilikalit aus Beispiel 3 wurden mit 5 g Methylcellulose trocken gemischt. Dieses Gemisch wurde im Kneter unter Zugabe von 95 ml Wasser verdichtet und bei einem Preßdruck von 30 bar zu Strängen mit 2 mm Durchmesser verarbeitet. Diese Stränge wurden über Nacht bei 110°C getrocknet und bei 500°C 5 h lang kalziniert. Die Seitendruckfestigkeit der Stränge ohne Binder betrug 9,5 N.

### Beispiel 5

100 g Titansilikalit aus Beispiel 3 wurden mit 5 g Methylcellulose trocken gemischt. Dieses Gemisch wurde im Kneter unter Zugabe von 70 ml Wasser und 12,5 g ammoniumstabilisiertem Kieselsol (Ludox® AS-40, DuPont, 40 Gew.-% SiO₂) verdichtet und bei einem Preßdruck von 30 bar zu Strängen mit 2 mm Durchmesser verarbeitet. Diese Stränge wurden über Nacht bei 110°C getrocknet und bei 500°C 5 h lang kalziniert. Die Seitendruckfestigkeit der Stränge mit 4,8 Gew.-% an Binder betrug 22,5 N.

### Beispiel 6

113 g geformter Titansilikalit gemäß Beispiel 5 wurden in ein Glasrohr mit eingearbeiteter Glasfritte eingefüllt und mit Glaswolle abgedeckt. Durch dieses gefüllte Glasrohr wurde nun eine Lösung aus 3,8 g PdCl₂ und 25 gew.-%iger wäßriger Ammoniaklösung langsam im Kreis gepumpt. Nach 24 h war die Kreislaufflüssigkeit an Palladium fast gänzlich abgereichert. Die Katalysatorstränge wurden im Glasrohr nun mit Wasser chloridfrei gewaschen. Danach wurden die so behandelten Katalysatorstränge bei 60°C im Vakuum 16 h lang getrocknet.

In einem Laborofen (Quarzglas, Durchmesser 10 cm, Länge der Heizzone 20 cm) wurden 50 g des Pd-modifizierten Produkts innerhalb von 90 min bei einer Temperatur von 50°C mit einer Gasmischung aus 20 l/h Stickstoff und 1 l/h Wasserstoff bei einer Drehzahl des Ofens von 50 U/min behandelt.

### Vergleichsbeispiel A

Zur Imprägnierung von Titansilikalitpulver aus Beispiel 3 wurde zunächst mit 0,515 g Palladium(II)chlorid und 120 g Ammoniaklösung (25 Gew.-% in Wasser) unter Rühren bei Raumtemperatur eine fleischfarbene Lösung hergestellt. In einem Rundkolben wurden 60 g des frisch hergestellten Titansilikalits aus Beispiel 3 in 130 g deionisiertem Wasser suspendiert. Dazu gab man die Gesamtmenge der vorbereitenden Pd-Tetraamin-chloro-Komplexlösung und rührte für den Verlauf einer Stunde im Rotationsverdampfer bei Raumtemperatur und Normaldruck. Abschließend wurde die Suspension bei 90 bei 100°C unter Vakuum (5 bis 19 mbar) eingedampft. Das weiße Produkt wurde direkt zur Reduktion weiterverwendet.

In einem Laborofen (Quarzglas, Durchmesser 5 cm, Länge der Heizzone 20 cm) wurden 20 g des Pd-modifizierten Produkts innerhalb von 90 min bei einer Temperatur von 50°C mit einer Gasmischung aus 20 l/h Stickstoff und 1 l/h Wasserstoff bei einer Drehzahl des Ofens von 50 U/min reduziert.

### Vergleichsbeispiel B

In einem Glasdruckreaktor wurden in 1650 ml Methanol als Lösungsmittel 2 g Katalysator aus Vergleichsbeispiel A unter Rühren suspendiert. Bei 60°C und einem Druck von 5 bar wurde sodann ein Gasgemisch aus 5 l/h Propen, 0,25 l/h Wasserstoff, 1 l/h Sauerstoff und 0,5 l/h Stickstoff eingeleitet. Aus gaschromatographischer Analyse fand man bei einem Abgasstrom von 6,8 l nach 44 h einen Volumenanteil an Propylenoxid von 0,30 %, nach 139 h einen Volumenanteil an Propylenoxid von 0,68 %, nach 270 h einen Volumenanteil an Propylenoxid von 0,50 % und nach 360 h einen Volumenanteil an Propylenoxid von 0,32 %. Dies belegt einen deutlichen Aktivitätsabfall innerhalb der letzten 220 h.

### Beispiel 7

In einem Glasreaktor wurden 9,8 g eines Katalysators aus Beispiel 6 eingebaut und in aufsteigender Fahrweise mit einem Lösungsmittelstrom von 4,5 kg/h einer wäßrigen Methanol-Lösung geflutet und im Kreis gepumpt. Am Reaktoreingang dosierte man druckgeregelt bei 5 bar 6,2 l/h Propen, 1,2 l/h Sauerstoff und 0,3 l/h Wasserstoff zu den 210 ml Lösungsmittel zu. Über eine Dauer von ca. 120 Stunden fand man im Abgas Konzentrationen an Propylenoxid von 0,11 Vol.-%.

## Patentansprüche

1. Oxidationskatalysator auf Basis von Titan- oder Vanadiumsilikaliten mit Zeolith-Struktur mit röntgenographischer Zuordnung zur MFI-, MEL- oder MFI/MEL-Mischstruktur und mit einem Gehalt von 0,01 bis 30 Gew.-% an einem oder mehreren Edelmetallen aus der Gruppe Ruthenium, Rhodium, Palladium, Osmium, Iridium, Platin, Rhenium, Gold und Silber, dadurch gekennzeichnet, daß der Oxidationskatalysator einem verfestigenden Formgebungsprozess durch Extrudieren zu Strängen unterworfen worden ist.

2. Oxidationskatalysator nach Anspruch 1, dadurch gekennzeichnet, daß er zu Strängen mit einem Durchmesser von 1 bis 10 mm extrudiert worden ist.

3. Oxidationskatalysator nach Anspruch 1 oder 2, dadurch gekennzeichnet, daß er bis zu 10 Gew.-% Bindemittel, bezogen auf die Gesamtmasse des geformten Katalysators, enthält.

4. Oxidationskatalysator nach Anspruch 3, dadurch gekennzeichnet, daß er als Bindemittel Verbindungen des Siliciums, Aluminiums, Bors, Phosphors, Zirkoniums und/oder Titans enthält.

5. Oxidationskatalysator nach den Ansprüchen 1 bis 4, dadurch gekennzeichnet, daß er zusätzlich ein oder mehrere Elemente aus der Gruppe Aluminium, Bor, Fluor, Zirkonium, Gallium, Zinn, Eisen, Kobalt und Nickel im Zeolithgerüst enthält.

6. Oxidationskatalysator nach den Ansprüchen 1 bis 5 mit einem molaren Verhältnis von Titan und/oder Vanadium zur Summe aus Silicium plus Titan und/oder Vanadium von 0,01 : 1 bis 0,1 : 1.

7. Oxidationskatalysator nach den Ansprüchen 1 bis 6, erhältlich durch Aufbringen der Edelmetalle in Form geeigneter Edelmetallkomponenten vor, während oder im Anschluß an den verfestigenden Formgebungsprozeß auf den Katalysator.

8. Verfahren zur Herstellung von Epoxiden aus Olefinen, Wasserstoff und Sauerstoff, dadurch gekennzeichnet, daß man die Olefine heterogenkatalytisch unter Verwendung eines Oxidationskatalysators gemäß den Ansprüchen 1 bis 7 umsetzt.

9. Verfahren zur Herstellung von Epoxiden nach Anspruch 8, dadurch gekennzeichnet, daß man die Umsetzung in einer Festbettapparatur durchführt.

10. Verfahren zur Herstellung von Propylenoxid aus Propen nach Anspruch 8 oder 9.

## Claims

1. An oxidation catalyst based on a titanium silicalite or vanadium silicalite having a zeolite structure assigned by X-ray diffraction to the MFI, MEL or MFI/MEL mixed structure, and containing from 0.01 to 30 % by weight of one or more noble metals selected from the group consisting of ruthenium, rhodium, palladium, osmium, iridium, platinum, rhenium, gold and silver, wherein the oxidation catalyst has been subjected to a compacting shaping process by extrusion to give extrudates.

2. An oxidation catalyst as claimed in claim 1, which has been extruded to give extrudates having a diameter of from 1 to 10 mm.

3. An oxidation catalyst as claimed in claim 1 or 2, which contains up to 10 % by weight, based on the total mass of the molded catalyst, of binder.

4. An oxidation catalyst as claimed in claim 3, which contains, as the binder, a compound of silicon, of aluminum, of boron, of phosphorus, of zirconium or of titanium.

5. An oxidation catalyst as claimed in any of claims 1 to 4, which additionally contains one or more elements selected from the group consisting of aluminum, boron, fluorine, zirconium, gallium, tin, iron, cobalt and nickel in the zeolite skeleton.

6. An oxidation catalyst as claimed in any of claims 1 to 5, having a molar ratio of titanium or vanadium to the sum of silicon and titanium or vanadium of 0.01:1 to 0.1:1.

7. An oxidation catalyst as claimed in any of claims 1 to 6, obtainable by applying the noble metals in the form of suitable noble metal components to the catalyst before, during or after the compacting shaping process.

8. A process for the preparation of an epoxide from an olefin, hydrogen and oxygen, wherein the olefin is converted under heterogeneous catalysis using an oxidation catalyst as claimed in any of claims 1 to 7.

9. A process as claimed in claim 8 for the preparation of an epoxide, wherein the reaction is carried out in a fixed-bed apparatus.

10. A process as claimed in claim 8 or 9 for the preparation of propylene oxide from propene.

## Revendications

1. Catalyseur d'oxydation à base de silicalites de titane ou de vanadium ayant une structure de zéolite avec une classification de structure mixte MFI, MEL ou MFI/MEL, obtenue par analyse aux rayons X, et ayant une teneur de 0,01 à 30% en poids en un ou plusieurs métaux nobles choisis dans le groupe formé par le ruthénium, le rhodium, le palladium, l'osmium, l'iridium, le platine, le rhénium, l'or et l'argent, caractérisé en ce que le catalyseur d'oxydation a été soumis à un processus durcissant de façonnage par extrusion en fils.

2. Catalyseur d'oxydation selon la revendication 1, caractérisé en ce qu'il a été extrudé en fils ayant un diamètre de 1 à 10 mm.

3. Catalyseur d'oxydation selon la revendication 1 ou 2, caractérisé en ce qu'il contient jusqu'à 10% en poids de liant, par rapport à la masse totale du catalyseur façonné.

4. Catalyseur d'oxydation selon la revendication 3, caractérisé en qu'il contient comme liant, des composés du silicium, de l'aluminium, du bore, du phosphore, du zirconium et/ou du titane.

5. Catalyseur d'oxydation selon les revendications 1 à 4, caractérisé en ce qu'il contient en plus un ou plusieurs éléments choisis dans le groupe formé par l'aluminium, le bore, le fluor, le zirconium, le gallium, l'étain, le fer, le cobalt et le nickel dans le squelette de zéolite.

6. Catalyseur d'oxydation selon les revendications 1 à 5, ayant un rapport molaire du titane et/ou du vanadium à la somme silicium + titane et/ou vanadium de 0,01:1 à 0,1:1.

7. Catalyseur d'oxydation selon les revendications 1 à 6, pouvant être obtenu en appliquant les métaux nobles sous forme de composants appropriés de métal noble sur le catalyseur avant, pendant ou après le processus durcissant de façonnage.

8. Procédé de préparation d'époxydes à partir d'oléfines, d'hydrogène et d'oxygène, caractérisé en ce que l'on met à réagir les oléfines avec catalyse hétérogène, en utilisant un catalyseur d'oxydation selon les revendications 1 à 7.

9. Procédé de préparation d'époxydes selon la revendication 8, caractérisé en ce que l'on réalise la réaction dans un appareil à lit fixe.

10. Procédé de préparation d'oxyde de propylène à partir du propène selon la revendication 8 ou 9.
